# EUROPEAN PATENT SPECIFICATION

(11) **EP 4 527 373 B1**
(45) Date of publication and mention of the grant of the patent: **24.09.2025**
(21) Application number: 24206976.3
(22) Date of filing: 30.07.2020
(51) Int. Cl.: A61K 9/00, A61K 9/20, A61K 31/40

(54) **NEW FORMULATIONS OF AMISULPRIDE**
NEUE FORMULIERUNGEN VON AMISULPRID
NOUVELLES FORMULATIONS D'AMISULPRIDE

(30) Priority: 31.07.2019 IT 201900013524
(43) Date of publication of application: 26.03.2025
(62) Divisional of application: 20764447.7
(73) Proprietor: Laboratori Baldacci S.p.A., 56124 Pisa (IT)
(72) Inventor: BALDACCI, Massimo, 56124 Pisa (PI) (IT)
(74) Representative: Dragotti & Associati S.R.L.

(56) References cited:
- WO-A1-2018/146490
- HANS RITTMANNSBERGER: "AMISULPRIDE AS AN AUGMENTATION AGENT IN TREATMENT RESISTANT DEPRESSION: A CASE SERIES AND REVIEW OF THE LITERATURE", PSYCHIATRIA DANUBINA, vol. 31, no. 2, 8 July 2019 (2019-07-08), Croatia, pages 148 - 156, XP055686604, ISSN: 0353-5053, DOI: 10.24869/psyd.2019.148
- ANONYMOUS: "PACKAGE LEAFLET: INFORMATION FOR THE USER", June 2018 (2018-06-01), pages 1 - 1, XP055687569, Retrieved from the Internet <URL:https://www.medicines.org.uk/emc/files/pil.3339.pdf> [retrieved on 20200420]
- ANONYMOUS: "IN USE PRODUCT SAFETY ASSESSMENT REPORT FOR LIQUID AMISULPRIDE", UKMI, March 2014 (2014-03-01), pages 1 - 4, XP055687570, Retrieved from the Internet <URL:https://www.ukmi.nhs.uk/filestore/ukmiaps/ProductsafetyassessmentforamisulprideliquidMar-2014.pdf> [retrieved on 20200420]

## Description

The object of the present invention are new, reduced dosage, solid or liquid formulations based on amisulpride or pharmaceutically acceptable salts thereof, for use in the treatment of dysthymia; in particular, the formulations of the present invention have the peculiarity of allowing a lower dosage of the active principle compared to the formulations known in the art, thus making it adjustable according to the specific characteristics of the patient.

### State of the art

Amisulpride is a drug belonging to the family of "substituted benzamides" which, similarly to the related compound sulpiride, is used in the treatment of various psychiatric disorders.

In particular, amisulpride works by acting as a selective antagonist on dopamine D2 and D3 receptors, showing a high affinity for these receptors.

The formulations of amisulpride currently available on the market are tablets that can be divided into halves having a content of 50 (DENIBAN and SULAMID, Sanofi Aventis), 100, 200, or 400 mg of amisulpride (SOLIAN, Sanofi Aventis, and corresponding generic products); or oral liquid formulations contained in 10 mL single-dose vials having a content of 50 mg of amisulpride (SOCIAN, Sanofi Aventis); or oral liquid formulations contained in a 60 mL graduated syringe and having an amisulpride concentration of 100 mg/mL (SOLIAN, Sanofi Aventis), which can be administered at a minimum dosage of 50 mg/dose; see also documents (5) and (7).

Hans Rittmannsberger et al. (6) describe the administration of amisulpride at dosages ranging from 25 to 100 mg, in order to verify efficacy and tolerability thereof in patients affected by resistant dysthymia and resistant major depression.

WO2018/146490A1 (8) describes the use of amisulpride for the treatment of vomiting and/or postoperative nausea.

Rein et al. (9) describe a clinical trial related to treatment of schizophrenia by administering amisulpride.

When administered in high doses, amisulpride has an anti-dopaminergic effect and acts as an atypical neuroleptic used in the treatment of psychosis, schizophrenia, and manic episodes in bipolar disorder. For this purpose, amisulpride is generally administered once a day, at daily doses normally between 100 and 400 mg.

While at lower dosages amisulpride causes an inhibitory block of pre-synaptic autoreceptors; this results in a facilitation of dopamine activity, and for this reason low dosages of amisulpride are used to treat major depression (in particular mixed forms, with anxiety and somatization disorders) and dysthymia (chronic, mild depression), being able to improve the patient's mood and determine a disinhibiting action, in particular facilitating interpersonal contact and involving a renewed interest of the subject for family and social environment. In such a case, the dose of amisulpride is normally of 50 mg per day.

Dosages of amisulpride lower than those normally used are advisable in the case of patients suffering from renal pathologies or in the case of elderly patients.

It has now surprisingly been found that, not only dosages amisulpride lower than those normally used (i.e. 50 mg/day) can still be used successfully in the treatment of major depression and/or dysthymia, but they can even be more effective while simultaneously reducing the drug side effects; in fact, it should be noted that amisulpride side effects are reported extremely frequently even using doses of the drug equal to 50 mg/day; see documents (1) to (4).

However, the formulations currently available on the market allow at most to reduce the drug dosage to 25 mg per administration, thus making it impossible to adjust the dose based on the disease characteristics, the individual patient specific response, and the reduction of the therapy side effects.

Providing the patient with an optimal formulation that allows the doctor and the patient to individualize therapy with *"ad personam"* daily doses even lower than 25 mg, or in any case intermediate between 50 and 25 mg, represents therefore an important therapeutic innovation that allows to exponentially increase the benefit/risk ratio of amisulpride in the treatment of major depression and dysthymia.

It is therefore apparent the need for new formulations and modes of administration that allow to use the drug at a lower daily dosage following a patient-specific dosage, while maintaining the therapeutic efficacy and side effects control, with an improvement in the benefit/risk ratio of the drug for the individual patient, especially in the treatment of dysthymia.

### Definitions

Unless otherwise defined, all terms of the art, notations, and other scientific terms used herein are intended to have the meanings commonly understood by those skilled in the art to which this description belongs. In some cases, terms with meanings that are commonly understood are defined herein for clarity and/or ready reference; therefore, the inclusion of such definitions in the present description should not be construed as being representative of a substantial difference with respect to what is generally understood in the art.

The terms "comprising", "having", "including", and "containing" are to be construed as open-ended terms (i.e., meaning "comprising, but not limited to"), and are to be considered as a support also for terms such as "consist essentially of", "consisting essentially of", "consist of", or "consisting of".

The term *"physiologically acceptable excipient"* refers to a substance devoid of any pharmacological effects of its own, and that does not produce adverse reactions when administered to a mammal, preferably a human being. Physiologically acceptable excipients are well known in the art and are described, for example, in Handbook of Pharmaceutical Excipients, sixth edition 2009.

The term *"pharmaceutically acceptable salts"* refers to those salts having the biological efficacy and properties of the salified or derivatized compound and which do not produce adverse reactions when administered to a mammal, preferably a human being. The pharmaceutically acceptable salts can be inorganic or organic salts; examples of pharmaceutically acceptable salts include, but are not limited to carbonate, hydrochloride, hydrobromide, sulfate, hydrogen sulfate, citrate, maleate, fumarate, trifluoroacetate, 2-naphthalenesulfonate, and para-toluenesulfonate. Further information on pharmaceutically acceptable salts may be found in Handbook of pharmaceutical salts, P. Stahl, C. Wermuth, WILEY-VCH, 127-133, 2008.

The term "elderly" or "elderly patient" refers to a person or a patient aged 65 years or more.

Depression, major depression, or dysthymia may be considered "treatment-resistant", when at least two therapies with antidepressants of different drug classes, which are adequate in dose, duration and compliance, fail to produce a significant clinical improvement, according to what commonly recognized in the psychiatric field; see for example (10) and (11).

### Figures

Figure 1. HDRS values from time T0 to time T3 during treatment with amisulpride with dosages ranging from 50 to 25 mg/day.
Figure 2. HDRS values from time T0 to time T4 during treatment with amisulpride with dosages ranging from 50 to 12.5 mg/day.

### Description

It has been surprisingly observed that the use of a daily dosage of amisulpride or pharmaceutically acceptable salts thereof in amounts of less than or equal to 25 mg of amisulpride or pharmaceutically acceptable salts thereof, allows an *"ad personam"* adjustment of the drug daily dosage and to optimize the risk/benefit ratio of the anti-dysthymic activity for each single patient, thus significantly increasing patient compliance.

Efficacy of amisulpride in the treatment of dysthymia was also surprisingly observed, even at dosages of less than 50 mg per day, i.e. at dosages commonly used with currently commercially available drugs and, in particular, at dosages equal to or less than 25 mg/day; it has also been observed that, in some patients, amisulpride may initially be effective at dosages equal to or greater than 50 mg/day and, subsequently, even at dosages of less than 50 mg/day and, in particular, at doses equal to or less than 25 mg/day.

In particular, the new solid or liquid formulations for use according to the present invention allow to administer the patient with amounts of amisulpride, or pharmaceutically acceptable salts thereof, from 0.1 to 12.5 mg, according to the specific needs and physiological characteristics of the patient.

The availability of the new liquid or solid formulations for use according to the present invention therefore facilitates the posology of amisulpride and allows its use as an anti-dysthymic drug, since the adjustment of the *"ad personam*" dosage with amounts of drug even lower than 50 mg/day allows to improve its efficacy in pro-dopaminergic, cortico-mesolimbic terms with a substantial increase in the benefit/risk ratio of the molecule also by virtue of the increased control of side effects.

An object of the present invention is therefore represented by a pharmaceutical formulation containing amisulpride and at least one physiologically acceptable excipient, said formulation being either (i) in a solid form divisible into fractions having an amisulpride content of less than or equal to 12.5 mg, preferably of between 0.1 and 12.5 mg, or (ii) in the form of an aqueous solution contained in a drop dispenser device, whose drops contain from 0.1 to 5 mg of amisulpride each, for use in the treatment of dysthymia.

According to an aspect of the invention, the above formulation is solid, preferably a tablet, and can be divided into fractions having a content of amisulpride, or pharmaceutically acceptable salts thereof, of less than or equal to about 12.5 mg, preferably between 0.1 and 12.5 mg, even more preferably between 2.5 and 12.5 mg; preferably, the aforesaid solid formulation can be divided into 2 or 4 fractions.

According to a further aspect of the invention, the formulation is liquid, i.e. it is an aqueous solution that can be administered in drops; for this purpose, it is contained in a suitable drop dispensing device. The above solution has a concentration of amisulpride, or pharmaceutically acceptable salts thereof, preferably of between 2.5 mg/mL and 100 mg/mL; even more preferably, it has a concentration of about 50 mg/mL. According to the invention, each drop contains from 0.1 to 5 mg of amisulpride or pharmaceutically acceptable salts thereof, preferably about 2.5 mg.

The physiologically acceptable excipients that can be used in the formulations for use according to the present invention can be selected from sweeteners, preservatives, stabilizing agents, anti-caking agents, acidity correctors or flavoring agents.

The aforementioned formulations are for use in the treatment of dysthymia; according to a further aspect of the invention, dysthymia is non-resistant.

Also disclosed is a drop dispensing device containing an aqueous solution of amisulpride or pharmaceutically acceptable salts thereof, said aqueous solution of amisulpride or pharmaceutically acceptable salts thereof having a concentration of between 2.5 mg/mL and 100 mg/mL, preferably a concentration of about 50 mg/mL.

The content by weight of amisulpride in the fractions of the fractionable formulations for use according to the present invention, or in the drops in case of liquid formulations, has a tolerance equal to ± 1%, preferably equal to ± 0.5%.

According to the present invention, the drop dispensing device is characterized in that each aqueous solution drop contains from 0.1 to 5 mg of amisulpride or pharmaceutically acceptable salts thereof, preferably each drop contains about 2.5 mg of amisulpride or pharmaceutically acceptable salts thereof.

Preferably, the aqueous solution of amisulpride or salts thereof for use according to the present invention comprises a sweetener selected from sodium saccharinate, sucrose, acesulfame K or other artificial or natural sweeteners, selected from steviol glycosides such as stevia glycosides or polyols such as sorbitol, a sequestering agent selected from gluconolactone or sodium gluconate, a preservative agent selected from methyl p-hydroxybenzoate, propyl p-hydroxybenzoate, potassium sorbate or sodium metabisulfite, natural or artificial flavoring agents that may contain ethanol or propylene glycol, selected from caramel flavor, sweet orange essential oil, vanillin, and apricot essence, acidity correctors, such as hydrochloric acid.

According to a further aspect, the aqueous solution of amisulpride for use according to the present invention may contain one or more physiologically acceptable co-solvents, such as for example alcohols, preferably ethanol.

Even more preferably, the aqueous solution of amisulpride for use according to the present invention comprises sodium saccharinate in an amount of between 1 and 3 g, gluconolactone in an amount of between 200 and 400 mg, sodium gluconate in an amount of between 25 and 75 mg, 3 M hydrochloric acid in an amount of between 0.1 and 5 mL, methyl p-hydroxybenzoate in an amount of between 25 and 75 mg, propyl p-hydroxybenzoate in an amount of between 10 and 40 mg, potassium sorbate in an amount of between 25 and 75 mg, caramel flavor in an amount of between 300 and 600 mg and water up to 50 mL.

In a preferred aspect, the drop dispensing device is characterized in that it delivers a volume from 0.02 to 0.2 mL of an aqueous solution of amisulpride or pharmaceutically acceptable salts thereof per drop, preferably from 0.05 to 0.1 mL per drop of an aqueous solution of amisulpride or pharmaceutically acceptable salts thereof.

In a further preferred aspect, the drop dispensing device is characterized in that it contains a volume of between 1 and 20 mL of an aqueous solution of amisulpride or pharmaceutically acceptable salts thereof, preferably of between 1 and 5 mL of an aqueous solution of amisulpride or pharmaceutically acceptable salts thereof, more preferably of 5 mL.

A further object is amisulpride for use in the treatment of dysthymia, characterized in that it is administered in drops containing an aqueous solution of amisulpride or pharmaceutically acceptable salts thereof having a concentration ranging between 2.5 mg/mL and 100 mg/mL, preferably of about 50 mg/mL.

According to an aspect, in the treatment of dysthymia 1 to 50 drops per day of solution of amisulpride or pharmaceutically acceptable salts thereof are administered, preferably 2 to 30 drops per day of aqueous solution of amisulpride or pharmaceutically acceptable salts thereof, more preferably 5 to 15 drops per day.

According to a further aspect, in the treatment of dysthymia, drops of an aqueous solution of amisulpride or pharmaceutically acceptable salts thereof are administered, which contain an amount of amisulpride or pharmaceutically acceptable salts thereof of between 0.1 and 5 mg per drop, preferably each drop contains about 2.5 mg of amisulpride or pharmaceutically acceptable salts thereof.

In the treatment of dysthymia, a dose of amisulpride or pharmaceutically acceptable salts thereof ranging from 2.5 to 50 mg per day can be administered for a period ranging from 30 to 90 days, more preferably a dose of amisulpride or pharmaceutically acceptable salts thereof ranging from 12.5 to 37.5 mg per day for a period of between 30 and 90 days. Even more preferably, multiples of 2.5 mg of amisulpride liquid solution are administered per day per patient.

Preferably, through administration of amisulpride by means of variable fractions of 2.5 mg it is possible to obtain dosages of 47.5 mg/day, 45 mg/day, 42.5 mg/day, 40 mg/day, 37.5 mg/day, 35 mg/day, 32.5 mg/day. day, 30 mg/day, 27.5 mg/day, 25 mg/day, 22.5 mg/day, 20 mg/day, 17.5 mg/day, 15 mg/day, 12.5 mg/day, 10 mg/day, 7.5 mg/day, 5 mg/day or 2.5 mg/day of amisulpride or pharmaceutically acceptable salts thereof.

This possibility of administering the drug has the advantage of allowing the doctor and the individual patient to find the *"ad personam"* dose having the best benefit/risk ratio for the patient.

The aqueous solution of amisulpride or pharmaceutically acceptable salts thereof is administered by using the drop dispenser device.

Patients suffering from dysthymia treated with amisulpride require dosages of amisulpride, or pharmaceutically acceptable salts thereof, of less than or equal to 50 mg/day, preferably said patients need dosages of amisulpride, or pharmaceutically acceptable salts thereof, of between 2.5 mg/day and 47.5 mg/day, more preferably said patients require dosages of amisulpride, or pharmaceutically acceptable salts thereof, of between 12.5 mg/day to 37.5 mg/day.

Even more preferably, said patients require dosages of amisulpride, or pharmaceutically acceptable salts thereof, as multiples of 2.5 mg/day.

In a further preferred aspect, amisulpride for use according to the present invention is administered at a dosage of less than 50 mg/day and by means of a dosage that can be divided into amounts ranging from 2.5 to 47.5 mg/day, by administration in drops. A further aspect is represented by amisulpride or pharmaceutically acceptable salts thereof for use in the treatment of dysthymia, wherein amisulpride is administered at a dosage of less than 25 mg/day.

According to the present invention, the pharmaceutical formulation containing amisulpride or pharmaceutically acceptable salts thereof is for use in the treatment of dysthymia, wherein amisulpride may be administered for a first period of time at a dosage equal to or greater than 50 mg/day and for a second period of time at a dosage of less than 50 mg/day; according to an aspect of the invention, during said first period of time amisulpride is administered at a dosage of 50 mg/day, while during said second period of time amisulpride is administered at a dosage equal to or less than 25 mg/day. In particular, said first period of time may have a maximum duration of 10 weeks, preferably 5 weeks, even more preferably 4 weeks; while said second period of time may have a minimum duration of 3 weeks, preferably 7 weeks, even more preferably 10 weeks.

The following examples are illustrative and not limitative of the invention; in particular, the aqueous solution according to the present invention can be formulated as shown in Examples 5-6, while the divisible tablet can be formulated as shown in Example 7.

### Experimental Section

### Example 1

In the psychiatry clinic of Turin hospital, the efficacy and safety of a treatment with amisulpride was evaluated at the consolidated dosage of 1 tablet of 50 mg/day, administered in the morning for 4 - 5 weeks to patients with dysthymia.

In addition to objective (rating scale) and subjective detection of the efficacy between time 0 (baseline) and time 1 (after 1 week of therapy) and time 2 (after 4 - 5 weeks of therapy), the adverse effects to the treatment that appeared during the trial were also evaluated.

The preliminary data collected on first patients seem to indicate an important anti-dysthymic activity at the end of the first week (decrease in the rating scale score and subjective evaluation) (time T1 versus time T0); a decrease in anti-dysthymic efficacy in a good percentage of patients treated between time T2 and Time T1, intended as a worsening of numerical values in the rating scales and subjective evaluation.

In the same period (between time T1 and time T2), the appearance of neuroendocrine side effects such as, for example, hyperprolactinemia, amenorrhea, and increase in body weight and appetite was also noted in a good percentage of patients.

These preliminary data seem to confirm an unknown aspect, namely the loss of efficacy of amisulpride in a good percentage of cases over time (after 4 - 5 weeks) with respect to the good results obtained already after a week of treatment, with an increase in side effects such as hyperprolactinemia, amenorrhea, increase in body weight and appetite, again between T1 and T2.

It is therefore plausible, although not initially predictable on the basis of the data available in the literature, that amisulpride therapeutic action can be modulated "downward", *i.e.* doses of the drug lower than the traditional ones could restore the initial dysthymic therapeutic effect.

Some of the patients undergoing the study have, in fact, halved the dosage (1/2 tablet/day = 25 mg) after 4 - 5 weeks of full dosage (50 mg), and again others have even achieved the therapeutic efficacy again together with side effects control by further decreasing the dose to 25 mg/day for 3 days a week.

This demonstrates that "there is an individual threshold" for each individual patient to activate the mesocorticolimbic dopaminergic activity, and that the same can be achieved by modulating the dose of the drug downwards.

A further clinical collection of data on dysthymic patients treated with a dose of amisulpride of 12.5 mg/day was therefore planned, in order to confirm that a daily administration of small doses of the drug can restore therapeutic activity in dysthymic patients who had shown a loss of efficacy and an increase in side effects over time while using the drug at the consolidated dosage for the treatment of dysthymia (*i.e.* 50 mg/day).

### Example 2

### Clinical study on efficacy of amisulpride in dysthymia to confirm the decrease in efficacy of a standard dose of 50 mg/day after 5 weeks of treatment and the increase in efficacy obtained in the same patients after reducing the daily dosage to 25 mg/day.

16 patients affected by dysthymia were treated with amisulpride at the standard dosage of 50 mg/day.

The Hamilton Depression Rating Scale (HDRS) median value at the start of the study (T0) was 22.19 ± 3.89 (moderate depression).

After 7 days of treatment (T1), the HDRS median values had significantly decreased to 8 ± 2.48 (p<0.00001), thus confirming the good efficacy profile of the standard dose in the first 7 days of treatment.

The patients were re-evaluated after 5 weeks of treatment (T2) and, surprisingly, the HDRS median values increased significantly (from 8 at T1 to 12.75 at T2, p<00001), thus indicating the decrease in efficacy of a standard dosage after 5 weeks of treatment.

Each patient had the amisulpride dosage decreased from 50 mg to 25 mg at T2 (after 5 weeks of treatment) and treatment was continued at this low dose for 3 weeks (from T2 to T3); at time T3 (after 3 weeks of treatment with 25 mg per day of amisulpride) the HDRS values significantly decreased from 12.75 at T2 up to 3.19 ± 0.83 at T3 (p<0.00001), confirming not only an increase in the safety profile of low dosage (25 mg per day), but also an unexpected increase in efficacy of the low dose in patients where the 50 mg/day dose had demonstrated a lack of efficacy after 5 weeks of treatment.

The data confirm the decrease in efficacy of amisulpride at the standard-of-care dosage (50 mg per day) after 5 weeks (from 8 to 12.75 T1 to T2) and, above all, the increase in efficacy (from 12.75 to 3.19 between T2 and T3) upon using half of the standard of care (25 mg per day), testified by transition from Mild Depression to No Depression after 3 weeks of treatment with a low dose of amisulpride, also considering the different limit of international validated HDRS values, reported below:

| **HDRS VALUE** | **DEPRESSION** |
|---|---|
| <7 | No Depression |
| 8 - 17 | Mild Depression |
| 18 - 24 | Moderate Depression |
| >25 | Severe Depression |

It was also found that the HDRS values after 3 weeks of low dose treatment (3.19 = No Depression) were the only ones by which each patient under treatment could be considered clinically free of dysthymia during the 8-week course of treatment with different dosages.

No side effects were observed under the treatment program during the 3 week-treatment with 25 mg of amisulpride per day.

To our knowledge, this is the first study highlighting the increase in the anti-dysthymic efficacy of amisulpride, obtained by reducing the dosage of amisulpride from 50 mg to 25 mg per day.

Table 1 shows the HDRS values for each patient at different times:
T0 = basal time, before using amisulpride;
T1 = 7 days after use of 50 mg/day of amisulpride;
T2 = after 5 weeks of amisulpride at 50 mg/day, and
T3 = after three weeks of amisulpride at 25 mg/day.

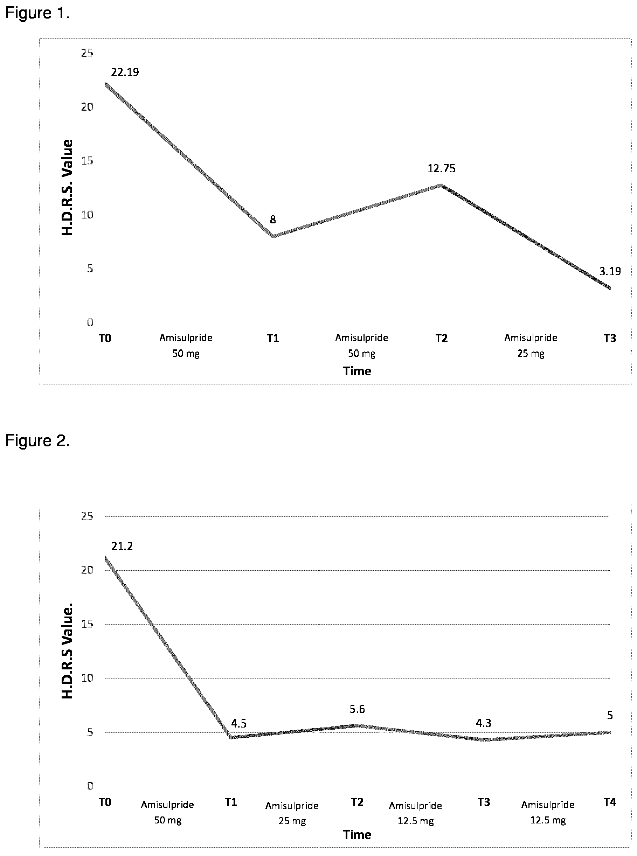

Such obtained values, shown also in Figure 1, confirm that:
- the decrease in efficacy of amisulpride at 50 mg/day after 5 weeks of treatment (T2 vs T0 and T1);
- the increase in efficacy following the switch from 50 to 25 mg/day of amisulpride (T3 vs T2 and T0).

**Table 1**

| **HDRS Values at times T0, T1, T2 e T3** | | | | |
|---|---|---|---|---|
| **Patient** | **T0** | **T1** | **T2** | **T3** |
| 1 | 21 | 11 | 14 | 3 |
| 2 | 22 | 7 | 13 | 4 |
| 3 | 15 | 4 | 11 | 4 |
| 4 | 25 | 8 | 12 | 4 |
| 5 | 18 | 6 | 13 | 2 |
| 6 | 29 | 13 | 15 | 3 |
| 7 | 21 | 9 | 14 | 4 |
| 8 | 26 | 6 | 10 | 2 |
| 9 | 27 | 4 | 11 | 3 |
| 10 | 23 | 11 | 16 | 3 |
| 11 | 18 | 7 | 11 | 2 |
| 12 | 25 | 8 | 13 | 2 |
| 13 | 19 | 10 | 16 | 3 |
| 14 | 25 | 7 | 13 | 4 |
| 15 | 18 | 9 | 9 | 4 |
| 16 | 23 | 8 | 13 | 4 |
| **AVERAGE VALUES** | **22.19** | **8.00** | **12.75** | **3.19** |
| **S.D.** | **3.89** | **2.48** | **2.02** | **0.83** |

| | | | | |
|---|---|---|---|---|
| T1 vs T0 = p<0.00001 (Highly significant efficacy) T2 vs T1 = p<0.00001 (Highly significant loss of efficacy) T3 vs T2 = p<0.00001 (Highly significant recovery of efficacy) | | | | |

### Example 3

### Clinical study on efficacy of amisulpride at 12.5 mg/day in 10 patients affected by dysthymia and previously treated with 50 and 25 mg/day dosages

10 patients affected by dysthymia were treated with amisulpride at a standard dose of 50 mg/day for 4 weeks (T0 to T1).

The median value of the HDRS values at the beginning of the study (T0) was 21.2 +/-3.55 (moderate depression).

After 4 weeks of treatment (T1) at the established standard dosage (50 mg/day) the median HDRS values decreased significantly to 4.5 ± 0.71 (p<0.00001) (no depression).

In order to control amisulpride side effects, all patients switched from 50 mg/day to 25 mg/day amisulpride at time T1 (after 4 weeks of treatment).

Patients were re-evaluated after 7 weeks of treatment from T0 and after 3 weeks of treatment from T1 to T2, and the median HDRS values remained in the "no depression" range (HDRS = 5.6 +/- 1.51).

At time T2, the daily dosage of amisulpride was reduced to 12.5 mg per day.

The HDRS values were evaluated at time T3 (after 9 weeks of treatment from T0 and after 2 weeks of treatment from T2): surprisingly, they had decreased from 5.6 (T2) to 4.3 (T3) only with reduction of amisulpride dosage from 25 mg to 12.5 mg per day.

Each patient continued the treatment with the extremely low dose of amisulpride (12.5 mg) from T3 to T4 (2 weeks of treatment) and, surprisingly, the HDRS value still remained in the "no depression" range (HDRS 5 ± 2.16 at time T4, after 11 weeks of treatment from T0 and after 4 weeks with amisulpride at 12.5 mg per day).

During the 4 weeks of treatment with amisulpride at 12.5 mg per day, no side effects due to the treatment were noted.

The data emerging from these results are truly surprising and unexpected, as this is the first collection of clinical data demonstrating the effectiveness of a low and unused daily dosage of amisulpride (12.5 mg/day).

Until now, no reference has ever been made to the usefulness of a daily dosage of 12.5 mg of amisulpride in the control of dysthymia, and demonstration that this low dosage could control symptoms in dysthymic patients without adverse events even after a period of treatment of 4 weeks, may confirm the usefulness of this very low, unused dosage for the treatment of dysthymia.

The switch from 50 to 25 mg and, surprisingly, from 25 to 12.5 mg showed no "decrease in efficacy" of the drug in dysthymic patients, confirming "the need to reduce the dosage" in some patients to obtain the best personalized dosage of amisulpride able, on the one hand, to control the patient's symptoms, and on the other hand to have a reduction in drug-related adverse reactions.

From published literature articles and most significant knowledge, there is no evidence of the use and relative efficacy of dosages of 12.5 mg per day; demonstration of the ability not only to reduce side effects but also to maintain clinical efficacy represent new knowledge for the physician who could use the lower dosage not only to reduce side effects but also to control dysthymic symptoms.

No side effects due to the treatment program were observed during the 4 weeks with amisulpride at 12.5 mg per day.

HDRS values for each patient at different times, summarized in Table 3, are reported below:
- T0 = basal time, before using amisulpride;
- T1 = four weeks after use of 50 mg/day of amisulpride;
- T2 = three weeks of amisulpride at 25 mg/day (7 weeks from T0);
- T3 = two weeks after use of 12.5 mg/day of amisulpride (9 weeks from T0), and
- T4 = four weeks after use of 12.5 mg/day of amisulpride (11 weeks from T0).

The results obtained, shown in Table 2 and Figure 2, confirm that:
- efficacy of amisulpride at 12.5 mg/day in patients who switched from 50 to 25 mg/day and from 25 mg to 12.5 mg/day;
- maintenance of treatment efficacy (HDRS values) after 4 weeks of treatment with a very low-dosage regimen (12.5 mg/day).

**Table 2**

| **HDRS Values at times T0, T1, T2 and T3** | | | | | |
|---|---|---|---|---|---|
| **Patient** | **T0** | **T1** | **T2** | **T3** | **T4** |
| 1 | 25 | 4 | 3 | 3 | 5 |
| 2 | 21 | 4 | 4 | 2 | 5 |
| 3 | 24 | 5 | 7 | 6 | 3 |
| 4 | 17 | 5 | 5 | 7 | 4 |
| 5 | 28 | 6 | 5 | 5 | 9 |
| 6 | 20 | 4 | 6 | 4 | 7 |
| 7 | 18 | 4 | 5 | 2 | 5 |
| 8 | 18 | 4 | 7 | 4 | 5 |
| 9 | 22 | 4 | 6 | 4 | 1 |
| 10 | 19 | 5 | 8 | 6 | 6 |
| **AVERAGE VALUES** | **21,2** | **4.5** | **5.6** | **4.3** | **5** |
| **S.D.** | **3.55** | **0.71** | **1.51** | **1.70** | **2.16** |

| | | |
|---|---|---|
| HDRS | T1 vs T0 | p<0.00001 (Highly significant efficacy) |
| HDRS | T2 vs T1 | p<0.051 (Difference in efficacy at the limits of significance) |
| HDRS | T3 vs T2 | p<0.087 (Difference in efficacy not significant) |
| HDR-s | T4 vs T3 | p<0.43 (Difference in efficacy not significant) |

**Table 3. Mode of Treatment**

| TIME | DURATION | SCHEDULED TREATMENT |
|---|---|---|
| T0--------T1 | 4 weeks | Amisulpride at 50 mg |
| T1 --------T2 | 3 weeks | Amisulpride at 25 mg |
| T2--------T3 | 2 weeks | Amisulpride at 12.5 mg |
| T3--------T4 | 2 weeks | Amisulpride at 12.5 mg |

### Example 4

### Clinical study to verify efficacy and safety of amisulpride in the treatment of dysthymia at dosages of 50, 37.5, 25 and 12.5 mg/day

In this study, the incidence of side effects and the need for an *"ad personam"* dosage adjustment of therapy with amisulpride were assessed. 13 patients were treated for 3 months with 50 mg/day of amisulpride; at the end of this period (T0) the efficacy of the therapy (HDRS) and the presence of side effects were assessed. In the presence of adverse events to therapy, the daily dosage was reduced to 25 mg/day for a further month; at the end of this treatment period (T1), the dosage was lowered to 12.5 mg/day (in case of persistence of side effects and efficacy) or it was increased to 37.5 mg/day (in case of no side effects but lack of efficacy) for a further month (T2).

Table 4 shows the results for each patient, both in terms of efficacy (HDRS) and the presence or absence of side effects.

| **Table 4** | | | |
|---|---|---|---|
| **Patient** | **T0** | **T1** | **T2** |
| **1** | | | |
| Dosage | 50mg | 25mg | 37.5mg |
| Side effects | tremor, cramps | No side effects | No side effects |
| HDRS | 11 | 20 | 5 |

| **2** | | | |
|---|---|---|---|
| Dosage | 50mg | 25mg | 12.5mg |
| Side effects | hyperprolactinemia, gynecomastia | Persisting side effects | No side effects |
| HDRS | 9 | 9 | 5 |

| **3** | | | |
|---|---|---|---|
| Dosage | 50mg | 25mg | 12.5mg |
| Side effects | dystonia, tremor, difficulty walking | Persisting side effects | Side effects improvement |
| HDRS | 7 | 8 | 20 |

| **4** | | | |
|---|---|---|---|
| Dosage | 50mg | 25mg | 37.5mg |
| Side effects | motor impediment, tremor | Persisting side effects | motor impediment, tremor |
| HDRS | 7 | 22 | 8 |

| **5** | | | |
|---|---|---|---|
| Dosage | 50mg | 25mg | 37.5mg |
| Side effects | tremor, > weight, <libido | Side effects improvement | Back to T0 Side effects |
| HDRS | 7 | 18 | 7 |

| **6** | | | |
|---|---|---|---|
| Dosage | 50mg | 25mg | 12.5mg |
| Side effects | hyperprolactinemia, > weight, breast tenderness | Side effects improvement | Back to T0 Side effects |
| HDRS | 6 | 5 | 17 |

| **7** | | | |
|---|---|---|---|
| Dosage | 50mg | 25mg | 37.5mg |
| Side effects | > weight, < libido, hyperprolactinemia | Persisting side effects | Side effects improvement |
| HDRS | 6 | 21 | 7 |

| **8** | | | |
|---|---|---|---|
| Dosage | 50mg | 25mg | 12.5mg |
| Side effects | parkinsonism | Persisting side effects | Side effects improvement |
| HDRS | 7 | 8 | 4 |

| **9** | | | |
|---|---|---|---|
| Dosage | 50mg | 25mg | 37.5mg |
| Side effects | motor impediment, tremor | Persisting side effects | Persisting side effects |
| HDRS | 4 | 23 | 9 |

| **10** | | | |
|---|---|---|---|
| Dosage | 50mg | 25mg | 37.5mg |
| Side effects | motor impediment, tremor, cramps | Persisting side effects | Persisting side effects |
| HDRS | 9 | 20 | 9 |

| **11** | | | |
|---|---|---|---|
| Dosage | 50mg | 25mg | 37.5mg |
| Side effects | motor impediment, < libido | Side effects improvement | Side effects improvement |
| HDRS | 7 | 20 | 8 |

| **12** | | | |
|---|---|---|---|
| Dosage | 50mg | 25mg | 37.5mg |
| Side effects | tremor, > weight, muscle stiffness | Persisting side effects | Persisting side effects |
| HDRS | 6 | 17 | 7 |

| **13** | | | |
|---|---|---|---|
| Dosage | 50mg | 25mg | 12.5mg |
| Side effects | tremor, > weight | Persisting side effects | Side effects improvement |
| HDRS | 5 | 3 | 17 |

The analysis of table 4 shows that:
1) All patients treated for 3 months with 50 mg/day of amisulpride showed, at the end of treatment (T0), known side effects to therapy.
2) After 1 month of treatment at 25 mg/day (T1) a decrease/reduction/absence of side effects was observed, but without therapeutic efficacy (see HDRS scores), with the consequent need to increase the dosage to 37.5 mg; or a persistence of side effects, with maintenance of therapeutic efficacy (HDRS), with the consequent need to further reduce the dose to 12.5 mg.
3) At the end of treatment with the dosage of 37.5 mg/day or 12.5 mg/day (T2), was either observed an improvement in side effects but with lack of therapeutic efficacy or a persistence of side effects with therapeutic efficacy.

This study highlights the need to provide doctor and patient with a solid or liquid formulation of amisulpride, which allows to modulate the dosage, in a simple and rational way, below the dosage of 50 mg/day normally used up to now, according to the specific needs of each individual patient.

### Example 5

Some examples of divisible formulations according to the present invention are reported in the following tables.

**Table 5**

| **Components** | **Solution** |
|---|---|
| Amisulpride | 2.5 q (=50mg/mL) |
| Sodium saccharinate (75-85%) | 1.6 q |
| Gluconolactone | 364.6 mq |
| Sodium gluconate | 54.4 mg |
| 3M Hydrochloric acid | Up to pH 4.5 |
| Methyl p-hydroxybenzoate | 50.49 mg |
| Propyl p-hydroxybenzoate | 25.87 mq |
| Potassium Sorbate | 50.9 mg |
| Caramel flavor | 499.9 mg |
| Water | Up to 50 mL |

**Table 6**

| **Components** | **Laboratory Solution** |
|---|---|
| Amisulpride | 1 g (= 50mg/2.5mL) |
| Sodium saccharinate | 1 g |
| Sodium metabisulfite | 74 mg |
| 3M Hydrochloric acid | Up to pH 5 |
| Methyl p-hydroxybenzoate | 65 mq |
| Propyl p-hydroxybenzoate | 10 mg |
| 70% Sorbitol | 10 mq |
| Caramel flavor | 500 mg |
| Water | Up to 50 mL |

**Table 7**

| **Components** | **Solution** |
|---|---|
| Amisulpride | 0,5 g (= 50mg/5 mL) |
| Acesulfame K | 3.5 q |
| Gluconolactone | 330 mg |
| Sodium gluconate | 60 mg |
| 3M Hydrochloric acid | Up to pH 6 |
| Methyl p-hydroxybenzoate | 50 mg |
| Propyl p-hydroxybenzoate | 26 mq |
| Potassium sorbate | 51 mg |
| Vanillin | 350 mq |
| Water | Up to 50 mL |

### Example 6 - Solution in Drops

The product consists of an aqueous solution containing 50 mg of Amisulpride per mL and is contained in a yellow glass bottle for pharmaceutical use with a capacity of about 6 mL, equipped with a polyethylene undercap and dropper; the bottle is sealed with a "child resistant" polypropylene cap.

The bottle will contain 1 mL of solution which will be dispensed in about 20 drops, so that each drop contains 2.5 mg of amisulpride.

The complete qualitative and quantitative composition per mL and per drop is shown in Table 8 below.

**Table 8**

| **Components** | **Components per mL** | **Components per drop** |
|---|---|---|
| Amisulpride | 50 mq | 2.5 mg |
| Sodium saccharinate (75-85%) | 32 mg | 1.6 mq |
| Gluconolactone | 7.29 mg | 0.365 mg |
| Sodium gluconate | 1.09 mg | 0.054 mg |
| 3M Hydrochloric acid | Up to pH 4.5 | Up to pH 4.5 |
| Methyl p-hydroxybenzoate | 1.01 mg | 0.050 mg |
| Propyl p-hydroxybenzoate | 0.52 mg | 0.026 mg |
| Potassium sorbate | 1.02 mg | 0.051 mg |
| Caramel flavor | 10 mg | 0.5 mg |
| Water | Up to 1 mL | Up to 0.05 mL |

### Example 7 - Divisible Tablet

The product consists of a 50 mg tablet of amisulpride, divisible into 2 or 4 parts. The tablet, weighing approximately 120 mg, is odorless and ivory-white in color, having a round shape and flat surface, with break scores on one side.

The qualitative and quantitative composition for each tablet and possible fractions thereof, obtainable using the break scores, is shown in Table 9 below.

**Table 9**

| **Components** | **Whole Tablet** | **½ Tablet** | **¼ Tablet** |
|---|---|---|---|
| Amisulpride | 50.0 mg | 25.0 mg | 12.5 mg |
| Isomalt | 48.5 mq | 24.25 mg | 12.125 mq |
| Microcrystalline cellulose | 20.0 mg | 10.0 mg | 5.0 mg |
| Sodium Croscarmellose | 1.0 mg | 0.5 mg | 0.25 mg |
| Magnesium stearate | 0.5 mg | 0.25 mg | 0.125 mg |

The tablets are packaged in opaque blisters consisting of PVC on one side and aluminum on the other.

### Bibliography

(1) Hyperprolactinemia with amisulpride. Indian J Psychiatry 2008 Jan - Mar; 50(1): 54 - 56.
(2) Hyperprolactinemia after low dose of amisulpride. Neuro Endocrinol Letter 2004 Dec 25 (6): 419 -22.
(3) A population approach to guide amisulpride dose adjustments in older patients with Alzheimer. The J of Clin Psych 2017; 28 (7) e844 - e851.
(4) Low dose amisulpride and elevation in serum prolactin. Letters to Editor. Journal of Clin Psychiat 2013 (Vol 74) N. 6.
(5) "Package leaflet: information for the user", June 2018, page1.
(6) Hans Rittmannsberger "Amisulpride as an augmentation agent in treatment resistant depression: a case series and review of the literature", Psychiatria Danubina. Vol. 31, n. 2 (2019), pagine 148-156.
(7) UKMi, "In use product safety assessment report for liquid amisulpride", March 2014, pagine 1-4.
(8) WO2018/146490A1.
(9) Rein et al. "Dimensions of psychopathology in schizophrenia: a factor analysis of the amisulpride database". European Neuropsycho pharmacology, Vol. 10 (2000), page 315.
(10) Alison Little, "Treatment-Resistant Depression", American Family Physician, July 15, 2009 ◆ Volume 80, Number 2, 167-172.
(11) Khalid Saad Al-Harbi, "Treatment-resistant depression: therapeutic trends, challenges, and future directions", Patient Preference and Adherence 2012:6 369-388.

## Claims

1. A pharmaceutical formulation containing amisulpride and at least one physiologically acceptable excipient, said formulation being either (i) in a solid form divisible into fractions having an amisulpride content of less than or equal to 12.5 mg or (ii) in the form of an aqueous solution contained in a drop dispenser device, whose drops contain from 0.1 to 5 mg of amisulpride each, for use in the treatment of dysthymia.

2. The formulation according to claim 1, **characterized in that** amisulpride is present as such or in the form of a pharmaceutically acceptable salt.

3. The formulation in solid form according to any one of the preceding claims, **characterized in that** it is a tablet.

4. The formulation in solid form according to any one of the preceding claims, **characterized in that** each fraction has an amisulpride content of between 0.1 and 12.5 mg.

5. The formulation in solid form according to any one of the preceding claims, **characterized in that** each fraction has an amisulpride content of between 2.5 and 12.5 mg.

6. The formulation in solid form according to any one of the preceding claims, **characterized in that** it is a divisible into 2 or 4 fractions.

7. The formulation in the form of an aqueous solution according to claim 1 or 2, **characterized in that** each drop contains 2.5 mg of amisulpride.

8. The formulation in the form of an aqueous solution according to claim 1 or 2, **characterized in that** it has an amisulpride concentration of between 2.5 mg/mL and 100 mg/mL

9. The formulation in the form of an aqueous solution according to claim 1 or 2, **characterized in that** it has an amisulpride concentration of 50 mg/mL.

10. The formulation in the form of an aqueous solution according to claim 1 or 2, **characterized in that** it contains one or more physiologically acceptable co-solvents, such as alcohols, preferably ethanol.

11. The formulation for use according to anyone of the preceding claims, wherein amisulpride is administered for a first period of time at a dosage equal to 50 mg/day and for a second period of time at a dosage of less than 50 mg/day.

12. The formulation for use according to claim 11, **characterized in that** during said second period of time amisulpride is administered at a dosage equal to or less than 37.5 mg/day, or at a dosage equal to or less than 25 mg/day, or at a dosage equal to or less than 12.5 mg/day.

13. The formulation for use according to anyone of claims 11 to 12, **characterized in that** said first period of time has a maximum duration of 10 weeks, preferably 5 weeks, even more preferably 4 weeks; and **in that** said second period of time has a minimum duration of 3 weeks, preferably 7 weeks, even more preferably 10 weeks.

14. Formulation for use according to any one of the preceding claims, **characterized in that** it is not administered to patients affected by renal pathologies and/or elderly patients; and/or **in that** said dysthymia is non-resistant.

## Patentansprüche

1. Pharmazeutische Formulierung, die Amisulprid und mindestens einen physiologisch verträglichen Hilfsstoff enthält, wobei die Formulierung entweder (i) in fester Form, die in Fraktionen mit einem Amisulpridgehalt von weniger als oder gleich 12,5 mg teilbar ist, oder (ii) in Form einer wässrigen Lösung, die in einer Tropfenabgabevorrichtung enthalten ist, deren Tropfen jeweils 0,1 bis 5 mg Amisulprid enthalten, zur Verwendung bei der Behandlung von Dysthymie vorliegt.

2. Formulierung nach Anspruch 1, **dadurch gekennzeichnet, dass** Amisulprid als solches oder in Form eines pharmazeutisch verträglichen Salzes vorhanden ist.

3. Formulierung in fester Form nach einem der vorstehenden Ansprüche, **dadurch gekennzeichnet, dass** es sich um eine Tablette handelt.

4. Formulierung in fester Form nach einem der vorstehenden Ansprüche, **dadurch gekennzeichnet, dass** jede Fraktion einen Amisulpridgehalt zwischen 0,1 und 12,5 mg aufweist.

5. Formulierung in fester Form nach einem der vorstehenden Ansprüche, **dadurch gekennzeichnet, dass** jede Fraktion einen Amisulpridgehalt zwischen 2,5 und 12,5 mg aufweist.

6. Formulierung in fester Form nach einem der vorstehenden Ansprüche, **dadurch gekennzeichnet, dass** sie in 2 oder 4 Fraktionen teilbar ist.

7. Formulierung in Form einer wässrigen Lösung nach Anspruch 1 oder 2, **dadurch gekennzeichnet, dass** jeder Tropfen 2,5 mg Amisulprid enthält.

8. Formulierung in Form einer wässrigen Lösung nach Anspruch 1 oder 2, **dadurch gekennzeichnet, dass** sie eine Amisulpridkonzentration zwischen 2,5 mg/ml und 100 mg/ml aufweist.

9. Formulierung in Form einer wässrigen Lösung nach Anspruch 1 oder 2, **dadurch gekennzeichnet, dass** sie eine Amisulpridkonzentration von 50 mg/ml aufweist.

10. Formulierung in Form einer wässrigen Lösung nach Anspruch 1 oder 2, **dadurch gekennzeichnet, dass** sie ein oder mehrere physiologisch verträgliche Co-Lösungsmittel, wie Alkohole, vorzugsweise Ethanol, enthält.

11. Formulierung zur Verwendung nach einem der vorstehenden Ansprüche, wobei Amisulprid während eines ersten Zeitraums in einer Dosierung von 50 mg/Tag und während eines zweiten Zeitraums in einer Dosierung von weniger als 50 mg/Tag verabreicht wird.

12. Formulierung zur Verwendung nach Anspruch 11, **dadurch gekennzeichnet, dass** während der zweiten Zeitspanne Amisulprid in einer Dosierung gleich oder weniger als 37,5 mg/Tag oder in einer Dosierung gleich oder weniger als 25 mg/Tag oder in einer Dosierung gleich oder weniger als 12,5 mg/Tag verabreicht wird.

13. Formulierung zur Verwendung nach einem der Ansprüche 11 bis 12, **dadurch gekennzeichnet, dass** die erste Zeitspanne eine maximale Dauer von 10 Wochen, vorzugsweise 5 Wochen, noch bevorzugter 4 Wochen, aufweist; und dass die zweite Zeitspanne eine Mindestdauer von 3 Wochen, vorzugsweise 7 Wochen, noch bevorzugter 10 Wochen, aufweist.

14. Formulierung zur Verwendung nach einem der vorstehenden Ansprüche, **dadurch gekennzeichnet, dass** sie nicht an Patienten mit Nierenerkrankungen und/oder an ältere Patienten verabreicht wird; und/oder dass die Dysthymie nicht resistent ist.

## Revendications

1. Formulation pharmaceutique contenant de l'amisulpride et au moins un excipient physiologiquement acceptable, ladite formulation étant soit (i) sous une forme solide divisible en fractions ayant une teneur en amisulpride inférieure ou égale à 12,5 mg ou (ii) sous la forme d'une solution aqueuse contenue dans un dispositif distributeur de gouttes, dont les gouttes contiennent de 0,1 à 5 mg d'amisulpride chacune, destinée à être utilisée dans le traitement de la dysthymie.

2. Formulation selon la revendication 1, **caractérisée en ce que** l'amisulpride est présent en tant que tel ou sous la forme d'un sel pharmaceutiquement acceptable.

3. Formulation sous forme solide selon l'une quelconque des revendications précédentes, **caractérisée en ce qu'**il s'agit d'un comprimé.

4. Formulation sous forme solide selon l'une quelconque des revendications précédentes, **caractérisée en ce que** chaque fraction a une teneur en amisulpride comprise entre 0,1 et 12,5 mg.

5. Formulation sous forme solide selon l'une quelconque des revendications précédentes, **caractérisée en ce que** chaque fraction a une teneur en amisulpride comprise entre 2,5 et 12,5 mg.

6. Formulation sous forme solide selon l'une quelconque des revendications précédentes, **caractérisée en ce qu'**elle est divisible en 2 ou 4 fractions.

7. Formulation sous la forme d'une solution aqueuse selon la revendication 1 ou 2, **caractérisée en ce que** chaque goutte contient 2,5 mg d'amisulpride.

8. Formulation sous la forme d'une solution aqueuse selon la revendication 1 ou 2, **caractérisée en ce qu'**elle a une concentration d'amisulpride comprise entre 2,5 mg/mL et 100 mg/mL.

9. Formulation sous la forme d'une solution aqueuse selon la revendication 1 ou 2, **caractérisée en ce qu'**elle a une concentration d'amisulpride de 50 mg/mL.

10. Formulation sous la forme d'une solution aqueuse selon la revendication 1 ou 2, **caractérisée en ce qu'**elle contient un ou plusieurs cosolvants physiologiquement acceptables, tels que les alcools, de préférence l'éthanol.

11. Formulation destinée à être utilisée selon l'une quelconque des revendications précédentes, dans laquelle l'amisulpride est administré pendant une première période de temps à un dosage égal à 50 mg/jour et pendant une seconde période de temps à un dosage inférieur à 50 mg/jour.

12. Formulation destinée à être utilisée selon la revendication 11, **caractérisée en ce que** pendant ladite seconde période de temps l'amisulpride est administré à une dose égale ou inférieure à 37,5 mg/jour, ou à une dose égale ou inférieure à 25 mg/jour, ou à une dose égale ou inférieure à 12,5 mg/jour.

13. Formulation destinée à être utilisée selon l'une quelconque des revendications 11 à 12, **caractérisée en ce que** ladite première période de temps a une durée maximale de 10 semaines, de préférence 5 semaines, plus préférablement encore 4 semaines ; et **en ce que** ladite seconde période de temps a une durée minimale de 3 semaines, de préférence 7 semaines, plus préférablement encore 10 semaines.

14. Formulation destinée à être utilisée selon l'une quelconque des revendications précédentes, **caractérisée en ce qu'**elle n'est pas administrée à des patients atteints de pathologies rénales et/ou à des patients âgés ; et/ou **en ce que** ladite dysthymie est non résistante.
